# EUROPEAN PATENT APPLICATION

(11) **EP 4 595 872 A1**
(43) Date of publication of application: **06.08.2025**
(21) Application number: 22965557.6
(22) Date of filing: 18.11.2022
(51) Int. Cl.: A61B 5/00, G16H 40/60, A61B 5/11, H04L 67/12, H04L 67/025

(54) **METHOD FOR PROCESSING MEDICAL PARAMETERS AND VIDEOS, MEDICAL SYSTEM, AND MEDICAL DISPLAY DEVICE**

(71) Applicant: Shenzhen Mindray Bio-Medical Electronics Co., Ltd., Shenzhen, Guangdong 518057 (CN)
(72) Inventor: WANG, Shengping, Shenzhen, Guangdong 518057 (CN); YANG, Ping, Shenzhen, Guangdong 518057 (CN); YANG, Kang, Shenzhen, Guangdong 518057 (CN)
(74) Representative: KIPA AB
(86) International application number: PCT/CN2022/132797
(87) International publication number: WO 2024/103376

(57) **Abstract**

A method for processing medical parameters and videos, a medical system, and a medical display device (100). The medical system comprises a camera apparatus, a medical device, a video processing server, a medical device server, and a workstation display device. The video processing server and the camera apparatus are in direct communication connection or indirect communication connection, and the video processing server is configured for analyzing video information to obtain and output a video analysis result of a target object. The medical device server is in direct communication connection or indirect communication connection with the medical device and the video processing server, respectively, and the medical device server is configured for acquiring medical parameter information and the video analysis result of the target object sent by the medical device; and performing fusion processing on the video analysis result and the medical parameter information of the target object to obtain and output a fusion result of videos and medical parameters. The workstation display device is in direct or indirect communication connection with the medical device server, and is configured for acquiring and displaying, on the same screen, the fusion result of videos and medical parameters.

## Description

### TECHNICAL FIELD

The disclosure generally relates to a technical field of medical device, and more specifically to a method for processing a medical parameter and video, a medical system, and a medical display device.

### BACKGROUND

Each patient in intensive care unit (ICU) usually uses multiple medical devices, including but not limited to a vital sign monitor, a ventilator, an infusion pump, etc. In order to centrally monitor a physiological state of a patient, a nurse station is generally further provided with a central station, which is connected with monitors of multiple beds in a department at the same time, and may also be simultaneously connected with monitors, ventilators, infusion pumps, etc. of multiple beds.

At the same time, in order to facilitate observation of patient, more and more ICUs have begun to configure each bed with an imaging apparatus, such as an imaging head, and deploy a terminal at the nurse station to simultaneously view real-time video images of multiple beds.

Simultaneously placing a video browsing terminal and a clinical workstation at the nurse station, not only takes up space, but also requires a clinician to manually find a video image of a corresponding bed on another screen if a patient state should be confirmed when seeing an alarm of a medical device. This is very inconvenient and prone to error.

### SUMMARY

This disclosure is proposed to solve at least one of above problems.

Specifically, a first aspect of this disclosure provides a medical system, including: an imaging apparatus, a medical device, a video processing server, a medical device server, and a workstation display device;
the imaging apparatus is configured to acquire video information of a target object;
the medical device is configured to acquire medical parameter information of the target object;
the video processing server is in direct or indirect communicative connection with the imaging apparatus, and is configured to obtain the video information of the target object which is acquired by the imaging apparatus, and analyze the video information, so as to obtain and output a video analysis result of the target object;
the medical device server is in direct or indirect communicative connection with the medical device and the video processing server respectively, wherein the medical device server is configured to: obtain the medical parameter information of the target object which is transmitted by the medical device, obtain the video analysis result of the target object which is outputted by the video processing server, and fuse the video analysis result of the target object and the medical parameter information of the target object, so as to obtain and output a fusion result;
the workstation display device is in direct or indirect communicative connection with the medical device server, and is configured to obtain and display the fusion result on a same screen.

A second aspect of this disclosure provides a medical system, including: an imaging apparatus, a medical device, a video processing server, a medical device server, and a workstation display device;
the imaging apparatus is configured to acquire video information of a target object;
the medical device is configured to acquire medical parameter information of the target object;
the video processing server is in direct or indirect communicative connection with the imaging apparatus, and is configured to obtain the video information of the target object which is acquired by the imaging apparatus, and analyze the video information, so as to obtain and output a video analysis result of the target object;
the medical device is in direct or indirect communicative connection with the video processing server, and is configured to obtain the video analysis result of the target object which is outputted by the video processing server, and fuse the video analysis result of the target object and the medical parameter information of the target object, so as to obtain and output a fusion result;
the medical device server is in communicative connection with the medical device, and is configured to obtain the fusion result which is forwarded by the medical device;
the workstation display device is in direct or indirect communicative connection with the medical device server, and is configured to obtain and display the fusion result on a same screen.

A third aspect of this disclosure provides a medical system, including: an imaging apparatus, a medical device, a medical device server and a workstation display device;
the medical device is configured to acquire medical parameter information of a target object;
the imaging apparatus is configured to acquire video information of the target object;
one of the medical device and the imaging apparatus is further configured to analyze the video information, so as to obtain and output a video analysis result of the target object;
the medical device server is in direct communicative connection with the medical device and the imaging apparatus respectively, or the medical device server is in indirect communicative connection with the imaging apparatus; wherein the medical device server is configured to fuse the video analysis result of the target object and the medical parameter information of the target object, so as to obtain and output a fusion result; or the medical device is configured to fuse the video analysis result of the target object and the medical parameter information of the target object, so as to obtain and output a fusion result, and transmit said fusion result to the medical device server;
the workstation display device is in direct or indirect communicative connection with the medical device server, and is configured to obtain and display the fusion result on a same screen.

A fourth aspect of this disclosure provides a medical system, including: an imaging apparatus, a medical device, a medical device server and a workstation display device;
the medical device is configured to acquire medical parameter information of a target object;
the imaging apparatus is configured to acquire video information of the target object; wherein the imaging apparatus is in communicative connection with the medical device;
the medical device server is in direct communicative connection with the medical device, and is configured to obtain the medical parameter information of the target object which is transmitted by the medical device;
the workstation display device is in direct or indirect communicative connection with the medical device server, and is configured to: obtain the video information of the target object which is acquired by the imaging apparatus, analyze the video information to obtain a video analysis result of the target object, obtain the medical parameter information of the target object which is transmitted by the medical device server, and fuse the video analysis result of the target object and the medical parameter information of the target object, so as to obtain and display a fusion result on a same screen.

A fifth aspect of this disclosure provides a method for processing a medical parameter and video, including:
acquiring video information of a target object;
acquiring medical parameter information which is outputted by a medical device which device corresponds to the target object;
after a target event is identified to occur in the video, correspondingly storing the medical parameter information within a preset time period before or before and after the target event occurs, the video information when the target event occurs, and the target event.

A sixth aspect of this disclosure provides a medical display device, including a communication interface, a display apparatus, and a processor; wherein:
the communication interface is configured to obtain medical parameter information and video information of at least one target object;
the processor is configured to:
   control the display apparatus to display an observation interface, wherein the observation interface includes at least one display area, wherein each display area correspondingly displays at least part of the medical parameter information of one target object;
   when a selection instruction for one of multiple display areas is obtained, control the display apparatus to display a detail interface of a selected target object which target object corresponds to a selected display area;
   when a review instruction is obtained, control the display apparatus to display a review interface of the selected target object, wherein the review interface is configured to display review information of the selected target object, wherein the review information includes the medical parameter information and the video information of the target object before a current moment, wherein the video information and the medical parameter information are associated with each other according to a timestamp.

According to the medical system of the first aspect to the fourth aspect of this disclosure, a fusion result is displayed on a same screen at a workstation display device, so as to enable a user to more conveniently view and analyze a video and medical parameter according to the fusion result, thereby improving a view and analysis efficiency, and making it not easy to confuse videos and medical parameters of different target objects, thus avoiding the problem of wrong determination of patient state due to viewing incompatible data. At the same time, displaying on a same screen saves more space. Furthermore, data corresponding to a same target object have been integrated, which avoids a user from searching for corresponding video information based on medical parameter information alone, or searching for corresponding medical parameter information based on video information alone, which is less prone to errors.

According to the method for processing a medical parameter and video of the fifth aspect of this disclosure, after identifying an occurrence of a target event in a video, medical parameter information within a preset time period before or before and after the target event occurs, and the video information when the target event occurs, are correspondingly stored with the target event. This can make it more convenient for a user to review the target event, the medical parameter information within a preset time period before or before and after the target event occurs, and the video information when the target event occurs, which helps a user to analyze data. When a false alarm occurs due to the fact that the target event affects a precise measurement of a medical parameter of a medical device, a user can determine whether the alarm is a false alarm caused by a body movement of patient through the video information when the target event occurs, so as to facilitate the user to temporarily block some possible false alarms and reduce alarm fatigue.

According to the medical display device of the sixth aspect of this disclosure, review information of a selected target object can be displayed, which review information includes medical parameter information and video information of the target object before a current moment, wherein the video information and the medical parameter information are associated with each other according to a timestamp, so as to enable the user to more conveniently view and analyze the video information and medical parameter information, thereby improving a view and analysis efficiency and being less prone to errors. In addition, both the medical parameter information and the video information are displayed through the medical display device. Compared with a method in which the video information is displayed separately through the video browsing terminal and the medical parameter information is separately displayed through another clinical workstation, this disclosure can save space. When a medical device alarm occurs, the user does not need to manually search for video information of a corresponding target object on another screen, and can confirm a patient state through the video information displayed on the medical display device. It is more convenient, quicker, and less prone to errors.

### BRIEF DESCRIPTION OF THE DRAWINGS

In order to provide a clearer explanation of technical solutions in embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be obtained based on these drawings without any creative effort.
FIG. 1 is a block diagram of a conventional medical system of this disclosure.
FIG. 2 is a block diagram of a medical system in an embodiment of this disclosure.
FIG. 3 is a block diagram of a medical system in an embodiment of this disclosure.
FIG. 4 is a diagram showing a corresponding relationship between a medical device and an imaging apparatus in an embodiment of this disclosure.
FIG. 5 is a block diagram of a medical system in an embodiment of this disclosure.
FIG. 6 is a block diagram of a medical system in an embodiment of this disclosure.
FIG. 7 is a block diagram of a medical system in an embodiment of this disclosure.
FIG. 8 is a block diagram of a medical system in an embodiment of this disclosure.
FIG. 9 is a block diagram of a medical system in an embodiment of this disclosure.
FIG. 10 is a flow chart of a method for processing a medical parameter and video in an embodiment of this disclosure.
FIG. 11 is a diagram showing a review interface in an embodiment of this disclosure.
FIG. 12 is a diagram showing a review interface for reviewing video information in an embodiment of this disclosure.
FIG. 13 is a diagram of a medical display device in an embodiment of this disclosure.
FIG. 14 is a diagram showing a display observation interface with multiple display areas in an embodiment of this disclosure.
FIG. 15 is a diagram showing each display area is provided with a video access button in an embodiment of this disclosure.
FIG. 16 is a diagram showing a target event and medical parameter information are displayed in a same display area in an embodiment of this disclosure.
FIG. 17 is a diagram showing an alarm event list in an embodiment of this disclosure.
FIG. 18 is a diagram showing detailed information and video information of a first alarm event is displayed in an embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the embodiments described are only some of the embodiments of this disclosure, not all of them. It should be understood that this disclosure is not limited by the embodiments described here. Based on the embodiments described in this disclosure, all other embodiments obtained by those skilled in the art without creative work, fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "including", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

Each patient in intensive care unit (ICU) usually uses multiple medical devices, including but not limited to a vital sign monitor, a ventilator, an infusion pump, etc. In order to centrally monitor a physiological state of a patient, a nurse station is generally also provided with a central station (that is, clinical workstation), which is connected with monitors of multiple beds in a department at the same time, and may also be simultaneously connected with monitors, ventilators, infusion pumps, etc. of multiple beds.

At the same time, in order to facilitate observation of patient, more and more ICUs have begun to configure each bed with an imaging apparatus, such as an imaging head, and deploy a video browsing terminal at the nurse station to simultaneously view real-time video images of multiple beds.

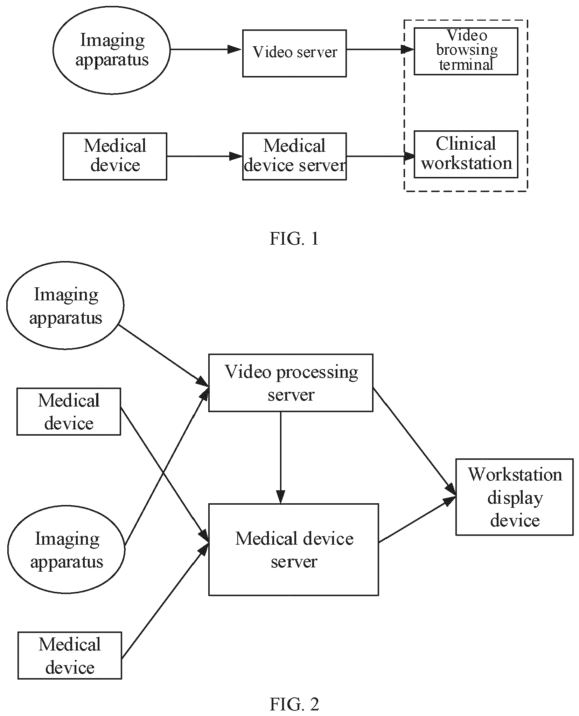

In a current medical system, as shown in FIG. 1, video images acquired by an imaging apparatus are transmitted to a video server, which stores the video images and forwards them to a video browsing terminal. The medical parameter information acquired by the medical device is transmitted to a medical device server, stored and forwarded to a clinical workstation by the medical device server (such as a central station). The video browsing terminal and the clinical workstation are respectively implemented through independent display devices. Simultaneously placing a video browsing terminal and a clinical workstation at the nurse station, not only takes up space, but also requires a clinician to manually find a video image of a corresponding bed on another screen (that is, a screen of the video browsing terminal) if a patient state should be confirmed when seeing an alarm of a medical device. This is very inconvenient and prone to error.

A body movement of the patient will affect an accurate measurement of medical device, resulting in some false alarms, which will cause medical staff to frequently run back and forth between multiple beds to deal with false alarms. Therefore, clinical medical staff hope to combine the body movement of the patient, so as to temporarily block some possible false alarms and reduce alarm fatigue.

In addition, an ICU patient may become agitated and, in severe cases, even be at risk of falling out of bed. If this risk is not discovered in time, it can have fatal consequences. If this risk can be discovered in time, it will help to reduce patient casualties caused by falling out of bed.

In view of at least one of above problems, this disclosure provides a method for processing a medical parameter and video, a medical system, and a medical display device, so as to at least partially solve the above problems.

Specifically, a method for processing a medical parameter and video, a medical system, and a medical display device of this disclosure are described in detail below in conjunction with the accompanying drawings. In the absence of conflict, features of following embodiments and implementations may be combined with each other.

Below, a medical system in each embodiment of this disclosure is described with reference to the accompanying drawings.

As an example, as shown in FIG. 2, the medical system includes: an imaging apparatus, a medical device, a video processing server, a medical device server, and a workstation display device. Optionally, the medical system may also include other required device(s).

The imaging apparatus is configured to acquire video information and/or image information of a target object, and may also acquire audio information, etc., wherein, when multiple target objects exist, each target object may correspond to one or more imaging apparatuses. The imaging apparatus may be, for example, any apparatus with an image acquisition function, such as an imaging head.

The imaging apparatus is capable of being in communicative connection with other device(s) in the medical system through a wireless communication or wired communication manner, so as to transmit the acquired video information.

The medical device is configured to acquire medical parameter information of the target object. In one embodiment, the medical device may include at least one of: a monitoring device, an examination device, a treatment device, etc. The monitoring device is configured to acquire vital sign information of the target object connected with the monitoring device. The monitoring device includes but is not limited to a bedside monitor or a wearable monitor, etc. The examination device is configured to exam the target object and generate examination information of the target object. The examination device may be an imaging examination device. For example, the examination device includes at least one of an ultrasonic device and an X-ray imaging apparatus, or any other suitable examination device. The treatment device is configured to treat the target object and output treatment information. Optionally, the treatment device includes at least one of a ventilator, a defibrillator, an infusion pump, an oxygen therapy device, an anesthesia machine, and an extracorporeal membrane oxygenation (ECMO), or other device that can treat a patient.

Optionally, the medical parameter information includes at least one of vital sign information, examination information, and treatment information. Usually, different medical devices measure and output different medical parameter information. For example, when the medical device is a monitoring device, the medical parameter information includes vital sign information. Optionally, the vital sign information includes at least one of: vital sign parameter information and alarm event information of the target object.

The vital sign parameter information includes, for example, data information of various vital sign parameters related to hemodynamics, and data information of some other basic parameters (i.e., a basic physiological parameter), such as HR (heart rate), PR (pulse rate), SpO2 (blood oxygen saturation), body temperature, RESP (respiratory rate), BP (blood pressure), ECG (electrocardiogram), etc., as well as includes trend graphs, waveform graphs or trend tables of various vital sign parameters.

Optionally, the examination information includes at least one of: medical imaging information, a medical test report, a medical diagnosis result, etc., or may also include other information related to the examination device. For example, when the examination device is an ultrasound device, the medical imaging information may include an ultrasound image, the medical test report may include an ultrasound test report, and the medical diagnosis result may include a diagnosis result made by doctor based on an ultrasound image.

In one example, the treatment information includes at least one of: medication administration information, ventilation parameter information, and defibrillation treatment parameter information; the medication administration information is, for example, medication administration information obtained from an infusion pump, such as a medication dosage, an infusion flow rate, etc. The ventilation parameter information is, for example, respiratory parameter information obtained from a ventilator, such as a pressure waveform, a volume waveform or a flow waveform, a tidal volume, a positive end-expiratory pressure (PEEP), and the likes. The defibrillation treatment parameter information is, for example, parameter information obtained from a defibrillator, such as a defibrillation time, an ECG parameter, etc.

The video processing server can be implemented by at least one server, and the video processing server is in direct or indirect communicative connection with the imaging apparatus. The video processing server is configured to obtain video information of the target object which is acquired by the imaging apparatus, analyze the video information, so as to obtain and output a video analysis result of the target object; wherein the video processing server can be any suitable device that can serve as a data transfer, such as a medical device corresponding to the same target object, for example a monitoring device. Exemplarily, the video processing server may also obtain real-time video information acquired by the imaging apparatus and forward said information, and may further store the obtained video information to facilitate a subsequent review of a user.

The video analysis result is obtained by identifying and processing the video information of each target object acquired by the imaging apparatus, and the video analysis result includes at least one of: facial appearance, movement posture of body part, the video processing server can identify and process the video information through any suitable intelligent algorithm to obtain the video analysis result of the target object. The movement posture of body part includes various possible movement postures that the body of the patient can perform, such as sitting, standing, turning over, falling out of bed, and movement of a body part (such as irregular movement of hand, lifting leg(s), shaking head, raising head, etc.).

In some embodiments, when the video processing server simultaneously accesses multiple imaging apparatuses respectively corresponding to multiple target objects, the multiple imaging apparatuses have their own identification information. For example, the identification information can be a code assigned to each imaging apparatus according to a channel, and can provide an interface for accessing real-time video information and historical data (such as historical video information) acquired by said imaging apparatus according to said channel, wherein the historical video information is video information before a current moment. Each imaging apparatus may also be identified by any other suitable identification information.

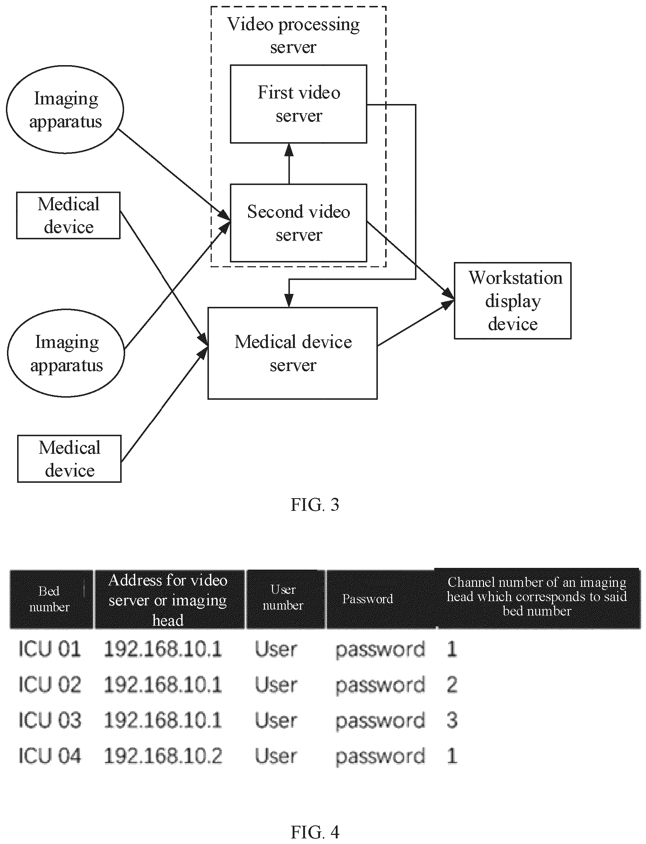

In some embodiments, as shown in FIG. 4, the video processing server may further include a first video server and a second video server, wherein the first video server is configured to obtain video information of a target object acquired by the imaging apparatus, analyze said video information, so as to obtain and output a video analysis result of the target object, and the second video server is configured to obtain video information of the target object through the imaging apparatus, and store and/or forward said video information. For example, the second video server and the imaging apparatus are in direct communicative connection, or the second video server is in indirect communicative connection with the imaging apparatus through the medical device. The second video server is in direct communicative connection with the first video server, and the second video server is configured forward the video information acquired by the imaging apparatus to the first video server, so as to enable the first video server to analyze and identify the video information.

The medical device server is in direct or indirect communicative connection with the medical device and the video processing server respectively. In some embodiments, as shown in FIG. 3, when the video processing server includes a first video server and a second video server, the first video server and the medical device server are in indirect communicative connection with each other through the medical device or the second video server, or the first video server and the medical device server are in direct communicative connection with each other.

The medical device server is configured to obtain the medical parameter information of the target object which is transmitted by the medical device, obtain the video analysis result of the target object outputted by the video processing server, and fuse the video analysis result of the target object and the medical parameter information of the target object, so as to obtain and output a fusion result.

In one example, fusing the video analysis result of the target object and the medical parameter information of the target object, so as to obtain and output a fusion result, including: based on a corresponding relationship between the medical device and the imaging apparatus, fusing the video analysis result of the target object and the medical parameter information of the target object, so as to obtain and output the fusion result, wherein the target object is associated with the medical device and the imaging apparatus which correspond to each other, wherein the fusion result includes at least one of: medical parameter information when an alarm event occurs, medical parameter information before an alarm event occurs or within a preset time period before and after an alarm event occurs, video information within a preset time period before and after an alarm event occurs, image information when an alarm event occurs, and video information when an alarm event occurs.

Optionally, the corresponding relationship between medical device and imaging apparatuses is stored in the medical device server or the video processing server. The corresponding relationship can be stored in a form of a list. For example, the corresponding relationship includes: position information of the medical device (such as represented by information of department bed number), an address of the video processing server or the imaging apparatus, a channel number of the imaging apparatus which apparatus corresponds to each bed, or further includes a username, a password, etc., as shown in FIG. 4. Through this corresponding relationship, the video analysis result of the target object and the medical parameter information of the target object can be fused, so as to obtain and output the fusion result; wherein the target object is associated with the medical device and the imaging apparatus which are corresponding with each other.

Optionally, alarm events for different medical devices are also different. For example, taking the medical device as a monitor, alarm event information of the monitor includes but is not limited to one or more of: alarm information for vital sign parameter, alarm information for device failure, technical alarm information, alarm information for artificial event, etc., wherein various alarm information may also include multiple alarms. For example, the alarm information for vital sign parameter includes alarm information when any parameter monitored by the monitor is abnormal, such as an HR high alarm and/or a PR high alarm, an HR low alarm and/or a PR low alarm, a low blood oxygen saturation alarm, etc.

For another example, when the medical device is a ventilator, alarm event information of the ventilator includes but is not limited to a respiration parameter alarm, a device abnormality alarm of ventilator, a system abnormality alarm of ventilator, an application scenario abnormality alarm of ventilator, etc., wherein the respiration parameter alarm includes but is not limited to a pressure alarm, a volume alarm, etc., wherein the pressure alarm includes but is not limited to an alarm for a too-high airway pressure, an alarm for a continuously high airway pressure, an alarm for a too-low airway pressure, an alarm for a pressure limit value, an alarm for a too-high positive end-expiratory pressure, an alarm for not reaching PinSP, an alarm for a too-low positive end-expiratory pressure, an alarm for a pressure limit value of a sigh cycle, etc., the volume alarm includes but is not limited to an alarm for a too-high ventilation volume per minute, an alarm for a too-low exhaled tidal volume, an alarm for a too-low ventilation volume per minute, an alarm for a capacity limit value, an alarm for a too-high exhaled tidal volume, an alarm for not reaching a tidal volume, etc.

The device abnormality alarm of ventilator can be an alarm triggered when any device of the ventilator is abnormal, such as an abnormality alarm for a pressure sensor, etc. The system abnormality alarm of ventilator also refers to an abnormality alarm of an operation system of the ventilator, such as system freeze, restart, etc. The application scenario abnormality alarm of ventilator includes but is not limited to an alarm for a pipeline disconnection, and other alarms triggered when some application scenarios are abnormal.

In some embodiments, the alarm event information also includes a target event identified in the video based on the video information, and the target event includes one of: the target object falling out of bed, the target object turning over, hand(s) of the target object moving irregularly, or other event(s) that may affect health of the target object.

When the medical device server is, for example, a central station with a display device, the medical device server can also display the fusion result, which corresponds to each target object. The medical device server can also store the fusion result to facilitate the user to review and analyze when needed. In some embodiments, the medical device server is further configured to store the medical parameter information, so as to facilitate a user to retrieve and review.

In some embodiments, in order to enable the medical device server to associate and fuse the acquired video information, video analysis result and medical parameter information according to the target object, that is, to associate and fuse the video information, video analysis result and medical parameter information which correspond to the same target object, the association and fusion are based on a timestamp, that is, the video information, video analysis result and medical parameter information which correspond to the same target object are associated with each other according to a timestamp, and configuration information is stored in the medical device server, wherein the configuration information includes a mapping relationship between identification information of the target object and identification information of the medical device, and a mapping relationship between the identification information of the target object and identification information of the imaging apparatus.

Optionally, the identification information of the target object includes but is not limited to one or more identification information, such as a bed number of the target object, a name of the target object, an identity of the target object (such as an identity card number (ID) or a medical insurance card number, etc.). The identification information of the target object can be used to associate the video information and the medical parameter information of the same target object to avoid mismatches that may cause a doctor to determine the medical condition of patient falsely.

The identification information of the medical device includes device identity code (ID) and/or device address, and the identification information of the imaging apparatus includes corresponding access address for video data or channel number of the imaging apparatus. The above identification information is only used as an example, and other suitable identification information can also be used in this disclosure.

The medical device server supports simultaneous access to medical devices of multiple beds (such as bedside medical devices). One bed can have one or more medical devices (such as a patient who needs a monitor to monitor a vital sign, a ventilator to maintain mechanical ventilation, an infusion pump to accurately administer medication(s), etc.). The medical device server supports classifying devices according to position information (same ward, same bed, same ambulance, etc.), or according to a patient (same patient).

For a scenario of classifying devices according to same position information, a configuration table is maintained at the medical device server. For example, this configuration table contains a corresponding relationship (also called mapping relationship) between the identification information of the target object, such as the medical position information (including but not limited to a bed number), and the identification information of the medical device, such as ICU 01 bed, a corresponding monitor address or device ID, a corresponding ventilator address or device ID, and a corresponding access address for video data of the imaging apparatus, as shown in Table 1.

**Table 1: Schematic table of a configuration table corresponding to devices classified according to same position information**

| Medical position information | Device information of monitor | Device information of respirator | Video information of imaging apparatus |
|---|---|---|---|
| ICU bed 01 | Device ID: PM1001 | Device ID: VENT1002 | rtsp:// name:pwd@ip:port/ channels/001 |
| | Device bed number: 01 | | |
| | | Device bed number: 01 | |
| ICU bed 02 | Device ID: PM1002 | Device ID: VENT1003 | rtsp:// name:pwd@ip:port/ channels/002 |
| | Device bed number: 02 | | |
| | | Device bed number: 02 | |

This configuration table can be maintained during installation. For example, the imaging apparatus is usually installed at the ceiling or hanging tower and cannot be moved. In this case, it can be configured during installation and does not need to be modified later.

In one embodiment, the medical device server is further configured to: in response to the medical device or the imaging apparatus being replaced, update the configuration information according to the identification information of a replaced medical device or the identification information of a replaced imaging apparatus, and the replacing includes but is not limited to adding a medical device or replacing another medical device for any target object. Specifically, for example, some medical device may also require dynamic maintenance during operation. For example, different ventilators may be selected according to a severity of the medical condition of patient. Therefore, the mapping relationship between the ventilator and the position information needs to be dynamically adjusted. This adjustment can be performed by clinical medical staff on the ventilator, such as manually changing the bed number information of the current ventilator, and can be performed by the server automatically; or, in some embodiments, the workstation display device is configured to obtain the identification information of the replaced medical device or the replaced imaging apparatus, and correspond said identification information to the identification information of the target object. The method for obtaining the identification information of the replaced medical device or the replaced imaging apparatus can be manual, for example, it can also be manually operated on the workstation to bind the ventilator to a new position (that is, updating the identification information of the ventilator into the configuration table where the identification information of the corresponding target object is located).

In other embodiments, at the medical device server, medical devices can also be classified according to a same patient. For example, when receiving a new patient, it is necessary to actively bind the medical device and the patient (i.e., the target object), wherein a binding relationship can be achieved by scanning a code. For example, the medical device server is configured to obtain the identification information of the target object (such as the ID of the target object) and the identification information of the medical device (such as the device ID) acquired and transmitted by a code scanning device, and store the identification information of the target object and the identification information of the medical device in a configuration table. The configuration table is maintained at the medical device server, as shown in Table 2.

The binding relationship between a patient and a device, for some fixed devices (such as an imaging apparatus, a monitor), the devices can also be bound to a position first (such as an imaging apparatus and a monitors are bound to ICU 01 bed). After the patient is bound with the position (ICU 01 bed), the binding relationship between the patient and the device at the corresponding position is automatically realized, that is, the identification information of the target object is associated with the identification information of the device which is associated with the target object.

**Table 2: Schematic table of a configuration table corresponding to devices classified according to a same patient**

| Patient information | Device information of monitor | Device information of respirator | Video information of imaging apparatus |
|---|---|---|---|
| PID: P1001 | Device ID : PM1001 | Device ID : VENT1002 | rtsp:// name:pwd@ip:port/ channels/001 |
| | | Device bed number: 01 | |
| | Device bed number: 01 | | |
| PID: P1002 | Device ID: PM1002 | Device ID: VENT1003 | rtsp:// name: pwd@ip:port/ channels/002 |
| | | Device bed number: 02 | |
| | Device bed number: 02 | | |

In one embodiment, the video processing server is configured to store the video information of the target object. The video processing server is further configured to store video configuration information. The video configuration information is for recording the bed information which corresponds to the target object at any time, and a corresponding access address for video data of the imaging apparatus. The time information, which corresponds to the video information, includes, for example, time information when the target object changes the bed. In this way, when the patient changes the bed during hospitalization, the video configuration information is maintained (for example, the video configuration information can be recorded in a form of a configuration table, as shown in Table 3). The video configuration information is for recording bed information which corresponds to the target object at any time, and a corresponding access address for video data of the imaging apparatus. The time information which corresponds to the video information, includes, for example, time information when the target object changes the bed, that is, historical bed change information is recorded. In this way, when medical staff retrieve the historical video information of any target object, they can browse complete video information, which is more convenient and quicker.

**Table 3: Schematic configuration table of video configuration information**

| Time | video information of imaging apparatus | Note |
|---|---|---|
| 5/01/2020 10:00:00 | rtsp:// name:pwd(@ip:port/channels/001 | At ICU bed 01 |
| 5/02/2020 11:00:00 | rtsp:// name:pwd@ip:port/channels/002 | The patient is transferred to ICU bed 02 at this moment |

The workstation display device is in direct or indirect communicative connection with the medical device server, and is configured to obtain and display the fusion result on a same screen. The fusion result is obtained and displayed on a same screen by the workstation display device, which enables a user to more conveniently view and analyze the video and medical parameter according to the fusion result, thereby improving a view and analysis efficiency, and making it not easy to confuse videos and medical parameters of different target objects, thus avoiding the problem of wrong determination of patient state due to viewing incompatible data. Furthermore, data corresponding to the same target object have been integrated, which avoids the user from searching for corresponding video information based on medical parameter information alone, or searching for corresponding medical parameter information based on video information alone, which is less prone to errors.

The workstation display device may be a display device of a workstation (also called a clinical workstation), an electronic medical record (referred to as EMR), a computer-based patient record (referred to as CPR), etc. Wherein, the medical device server is, for example, a central station. The central station can be, for example, one or more computer devices installed at a nurse station, and can have one or more display devices, which can be used to simultaneously display medical parameter information of one or more patients (also referred to as patient(s) or target object(s) in this disclosure), etc. The workstation can be configured to observe patient from different departments. The workstation can be connected with different central stations at the same time. Clinical managers can observe information of patients from different departments at a glance on the workstation, such as various medical parameter information, video information acquired by imaging apparatuses, etc.

Exemplarily, the first video server may also be in communicative connection with the workstation display device to transmit video information acquired by the imaging apparatus to the workstation display device for display. The video information may be real-time video information or historical video information stored.

In some embodiments, the video processing server is further configured to transmit the video analysis result to a workstation display device, and the workstation display device is configured to display, on a same screen, the video analysis result of the target object and the medical parameter information of the target object, wherein the target object is associated with the medical device and the imaging apparatus which correspond to each other. That is, the video analysis result and the medical parameter information of the same target object can be displayed on a same screen in real time, which is convenient for a user to view. When the video analysis result include a target event, the target event and the medical parameter information can also be displayed on a same screen. The target event can be marked by a preset display method to make it more eye-catching, so as to remind medical staff to provide timely help or treatment to the target object in the target event.

In some embodiments, the workstation display device can also obtain video information acquired by the imaging apparatus and medical parameter information acquired by the medical device, and display, on a same screen, the medical parameter information of the medical device and the video information, which respective information corresponds to a same target object. Wherein the workstation display device can be in direct or indirect communicative connection with the imaging apparatus. For example, for the indirect communicative connection, the workstation display device can be in communicative connection with the video processing server, the video processing server can be in communicative connection with the imaging apparatus. The video processor server receives real-time video information transmitted by the imaging apparatus, and forwards the real-time video information to the workstation display device. Optionally, the workstation display device can be in communicative connection with the medical device server, the imaging apparatus is in direct communicative connection with the medical device server, or the imaging apparatus is in communicative connection with the medical device server through the medical device (that is, the imaging apparatus and the medical device corresponding to the same target object), the medical device server transmits video information acquired by the imaging apparatus to the workstation display device, or the workstation display device can be in communicative connection with the imaging apparatus through the medical device, and the workstation display device is further configured to obtain video information transmitted by the medical device. In other embodiments, the workstation display device may be in direct communicative connection with the imaging apparatus, and the imaging apparatus transmits the acquired video information to the workstation display device for display.

In some embodiments, the workstation display device can be in communicative connection with the medical device server, which is further in communicative connection with the medical device. The workstation display device is configured to obtain the medical parameter information acquired by the medical device server, and display, on a same screen, the medical parameter information and the video information which respective information corresponds to the same target object. In other embodiments, the workstation display device is in communicative connection with the medical device, and the workstation display device can obtain the medical parameter information transmitted by the medical device, and display, on a same screen, the medical parameter information and the video information which respective information corresponds to the same target object, for example, displaying these information in a same display area, or displaying on a same screen video information based on the medical parameter information, in response to a view instruction to view a video, so as to facilitate a clinical user to better observe a state of the target object through the video information.

The medical parameter information and the video information, which respective information corresponds to the same target object, are displayed on a same screen through the workstation display device, so as to enable a user to view and analyze these two types of data more conveniently, thereby improving a view and analysis efficiency, and making it not easy to confuse videos and medical parameters of different target objects, thus avoiding the problem of wrong determination of patient state due to viewing incompatible data. Furthermore, data corresponding to a same target object have been integrated, which avoids a user from searching for corresponding video information based on medical parameter information alone, or searching for corresponding medical parameter information based on video information alone, which is less prone to errors.

In some embodiments, the video processing server and the medical device server may be implemented by a same server, or, when the video processing server includes a first video server and a second video server, at least two of the second video server, the first video server and the medical device server may be implemented by a same server.

In some embodiments, when the video processing server includes a first video server and a second video server; the workstation display device and at least two of the second video server, the first video server and the medical device server are implemented by a same computing device. Furthermore, the video processing server, the medical device server, and the workstation display device are implemented by a same computing device.

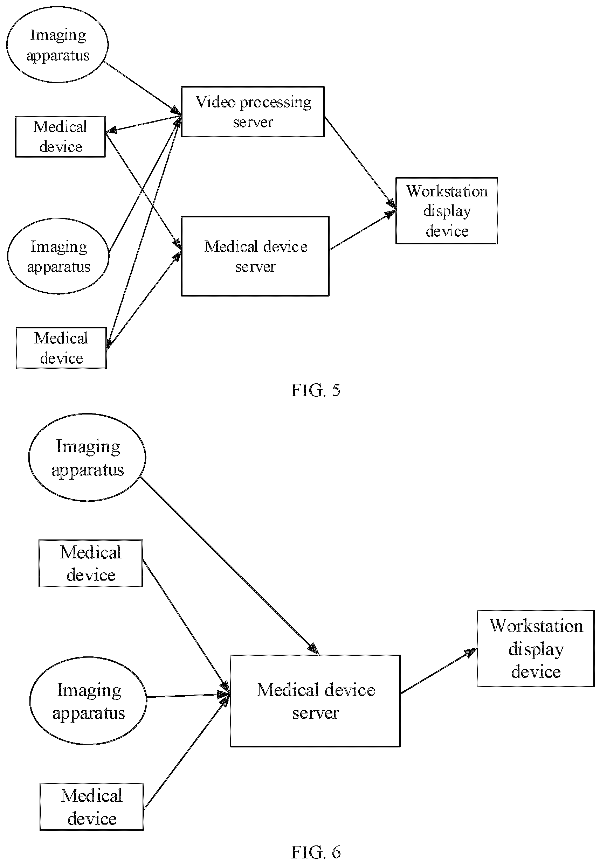

In another embodiment of this disclosure, a medical system is also provided, as shown in FIG. 5, wherein the medical system includes: a video processing server, an imaging apparatus, a medical device, a medical device server and a workstation display device.

The imaging apparatus is configured to acquire video information of a target object, and the medical device is configured to acquire medical parameter information of the target object; the video processing server is in direct or indirect communicative connection with the imaging apparatus, and the video processing server is configured to obtain the video information of the target object which is acquired by the imaging apparatus, analyze the video information, so as to obtain and output a video analysis result of the target object; the medical device is in direct or indirect communicative connection with the video processing server, and the medical device is configured to obtain the video analysis result of the target object outputted by the video processing server, and fuse the video analysis result of the target object and the medical parameter information of the target object, so as to obtain and output a fusion result; the medical device server is in communicative connection with the medical device, and is configured to obtain the fusion result forwarded by the medical device; the workstation display device is in direct or indirect communicative connection with the medical device server, and is configured to obtain and display the fusion result on a same screen.

Some details of this embodiment can be referred to the relevant description in the previous text, among which, the difference between this embodiment and the previous embodiment mainly lies in: the medical device is in direct or indirect communicative connection with the video processing server, and the medical device is configured to obtain the video analysis result of the target object outputted by the video processing server, and fuse the video analysis result of the target object and the medical parameter information of the target object, so as to obtain and output a fusion result. That is, the medical device fuses the data, and the corresponding medical device will also have some functions of the aforementioned medical device server, such as storing a configuration table that represents the corresponding relationship between the medical device and the imaging apparatus.

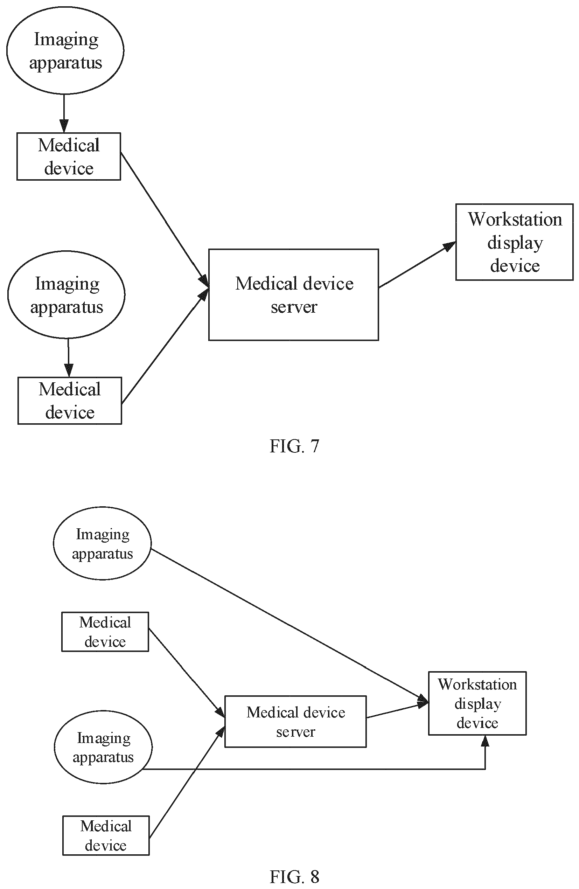

In yet another embodiment of this disclosure, a medical system is further provided, as shown in FIG. 6 and FIG. 7, including: an imaging apparatus, a medical device, a medical device server and a workstation display device.

The medical device is configured to acquire medical parameter information of a target object; the imaging apparatus is configured to acquire video information of the target object; one of the medical device and the imaging apparatus is further configured to analyze the video information, so as to obtain and output a video analysis result of the target object; the medical device server is in respective direct communicative connection with the medical device and the imaging apparatus, or the medical device server is in indirect communicative connection with the imaging apparatus, and the medical device server is configured to, as shown in FIG. 6, fuse the video analysis result of the target object and the medical parameter information of the target object, so as to obtain and output a fusion result, or, as shown in FIG. 7, the medical device is configured to fuse the video analysis result of the target object and the medical parameter information of the target object, so as to obtain and output a fusion result, and transmit said fusion result to the medical device server; the workstation display device is in direct or indirect communicative connection with the medical device server, and is configured to obtain and display the fusion result on a same screen.

Some details of this embodiment can be referred to the relevant description in the previous text, among which, the difference between this embodiment and the previous embodiment mainly lies in that the medical system of this embodiment does not have a video processing server, and one of the medical device and the imaging apparatus is further configured to analyze the video information, so as to obtain and output the video analysis result of the target object. The medical device server or the medical device fuses the video analysis result of the target object and the medical parameter information of the target object.

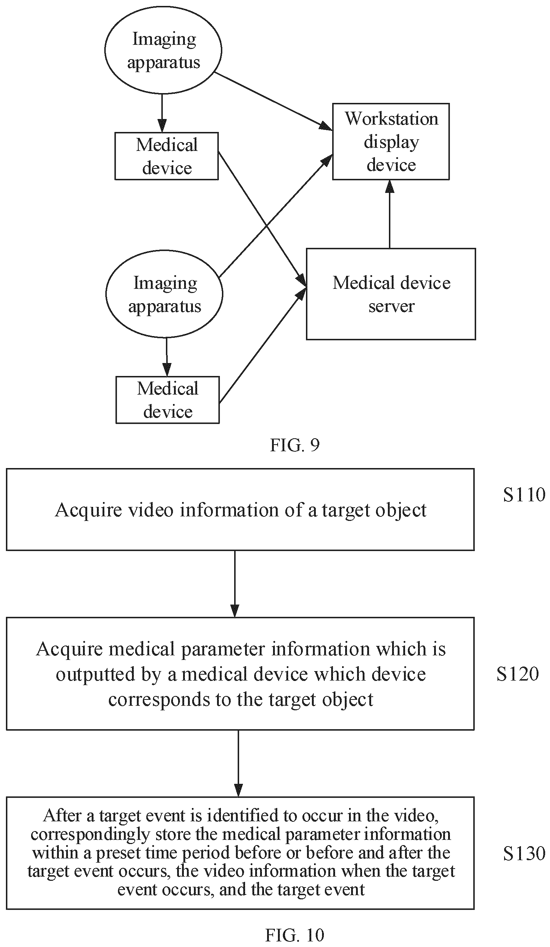

In another embodiment of this disclosure, a medical system is also provided, as shown in FIG. 8 and 9, the medical system includes: an imaging apparatus, a medical device, a medical device server and a workstation display device; the medical device is configured to acquire medical parameter information of a target object; the imaging apparatus is configured to acquire video information of the target object, the imaging apparatus is in communicative connection with the medical device, the medical device server is in direct communicative connection with the medical device, wherein the medical device server is configured to obtain the medical parameter information of the target object which is transmitted by the medical device; the workstation display device is in direct or indirect communicative connection with the medical device server, and in direct or indirect communicative connection with the imaging apparatus, wherein the workstation display device is configured to: obtain the video information of the target object which is acquired by the imaging apparatus, analyze the video information to obtain a video analysis result of the target object, obtain the medical parameter information of the target object which is transmitted by the medical device server, and fuse the video analysis result of the target object and the medical parameter information of the target object, so as to obtain and display a fusion result on a same screen.

Some details of this embodiment can refer to the relevant description in the previous text, among which, the main difference between this embodiment and the previous embodiment is that the medical system of this embodiment does not have a video processing server, and the workstation display device is configured to obtain the video information of the target object which is acquired by the imaging apparatus, analyze the video information to obtain the video analysis result of the target object, obtain the medical parameter information of the target object which is transmitted by the medical device server, and fuse the video analysis result of the target object and the medical parameter information of the target object, so as to obtain and display the fusion result on a same screen.

It is worth mentioning that when the imaging apparatus is in direct communicative connection with the workstation display device, the workstation display device can obtain real-time video information, but the workstation display device does not need to store the video information, so it will not have the function of video review. Of course, if the workstation display device stores the video information or the medical device server stores the video information, it can have the function of video review.

In summary, according to the medical system of this disclosure, a fusion result is displayed on a same screen of a workstation display device, so as to enable a user to more conveniently view and analyze a video and medical parameter according to the fusion result, thereby improving a view and analysis efficiency, and making it not easy to confuse videos and medical parameters of different target objects, thus avoiding the problem of wrong determination of patient state due to viewing incompatible data. Furthermore, the data corresponding to a same target object have been integrated, which avoids a user from searching for corresponding video information based on medical parameter information alone, or searching for corresponding medical parameter information based on video information alone, which is less prone to errors.

Furthermore, this disclosure also provides a method for processing a medical parameter and video, as shown in FIG. 10, the method including following steps.

Step S110, acquire video information of a target object.

Step S120, acquire medical parameter information which is outputted by a medical device which device corresponds to the target object.

Step S130, after a target event is identified to occur in the video, correspondingly store the medical parameter information within a preset time period before or before and after the target event occurs, the video information when the target event occurs, and the target event.

After identifying an occurrence of a target event in a video, medical parameter information within a preset time period before or before and after the target event occurs and the video information when the target event occurs are correspondingly stored with the target event. This can make it more convenient for a user to review the target event, the medical parameter information within a preset time period before or before and after the target event occurs, and the video information when the target event occurs, which helps a user to analyze data. When a false alarm occurs due to the fact that the target event affects a precise measurement of a medical parameter of a medical device, a user can determine whether the alarm is a false alarm caused by a body movement of patient through the video information when the target event occurs, so as to facilitate the user to temporarily block some possible false alarms and reduce alarm fatigue.

It is worth mentioning that correspondingly storing the medical parameter information within a preset time period before or before and after the target event occurs, the video information when the target event occurs, and the target event refers to that medical parameter information and video information when the target event occurs are stored corresponding to time information of the target event, such as based on a timestamp.

In one example, the processing method of this disclosure also includes: displaying, by a display apparatus, video information when a target event occurs and medical parameter information within a preset time period before or before and after the target event occurs. For example, the medical parameter information may be vital sign parameter information of the same target object monitored by a monitor, and wherein the vital sign parameter information includes waveform(s) and/or value(s), trend graph(s), etc. of vital sign parameter(s).

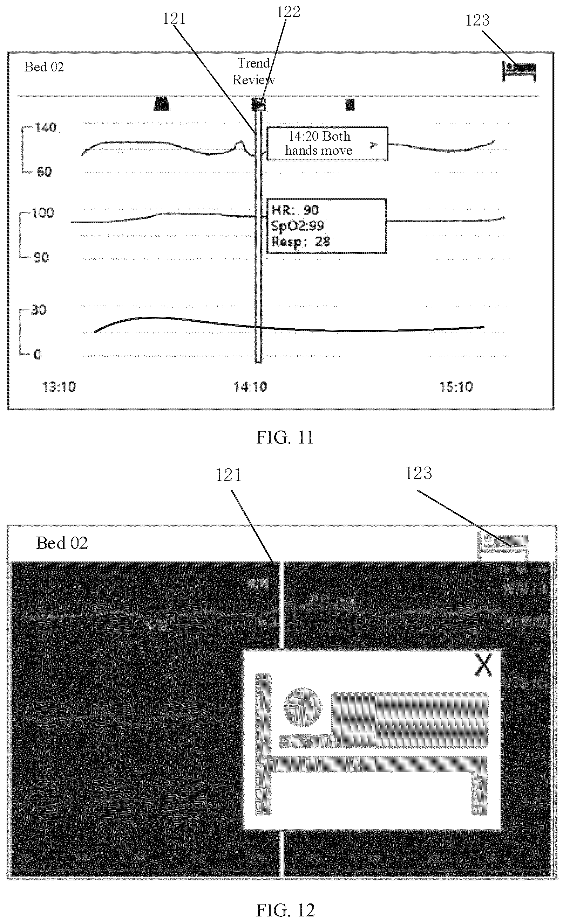

In some embodiments, the display apparatus provides a display observation interface. When the display apparatus is configured to display data information of multiple target objects, the display observation interface includes multiple display areas, each display area correspondingly displays at least part of the medical parameter information of one target object; the method of this disclosure also includes: when a selection instruction for one of the multiple display areas is obtained, controlling the display apparatus to display a detail interface of a selected target object which target object corresponds to a selected display area, wherein the detail interface is provided with a review button; and when a review instruction for review information within a preset time period is obtained, controlling the display apparatus to display corresponding review information on a review interface according to a time sequence within said preset time period. As shown in FIG. 11, the review interface displays a time indication which represents the time sequence, and a target event indication 122 is provided at a time point of the time indication which time point corresponds to the target event. In this way, the user can be clearly reminded that the target event has occurred to the target object at the time point indicated by the corresponding target event indication.

In some embodiments, as shown in FIG. 11, the method of this disclosure also includes: when a selection instruction for one target event indication is obtained, controlling the display apparatus to display event information of the target event which target event corresponds to a selected target event indication (for example, 14:20 both hands move) and the medical parameter information within a preset time period before or before and after the target event occurs (that is, data information of medical parameter, such as vital sign parameter information). Optionally, the selection instruction may be an instruction generated by moving a cursor to an occurrence time point of the target event, or an instruction generated by clicking the target event indication with a mouse, or other selection instruction for the target event indication. The event information includes but is not limited to at least one of: a text representation of the target event, time information when the target event occurs, and the likes. In some embodiments, the event information may also include video information or image information when the target event occurs, and/or video information within a preset time period before and after the target event occurs. By a combined display of the medical parameter information and the target event, it is convenient for the user to analyze an abnormality of the medical parameter information and determine whether the abnormality of the medical parameter information is caused by the target event, thereby more accurately determining the medical condition of patient.

In one embodiment, as shown in FIG. 11, the review interface also displays a video review button 123. The method of this disclosure further includes: in response to an operation instruction for the video review button 123, controlling the display apparatus to display on the review interface video information within a preset time period before and after the target event occurs.

In some embodiments, as shown in FIG. 12, the review interface also displays a cursor mark 121, and the method further includes: controlling the display apparatus to display the cursor mark 121 on the review interface; updating a position of the cursor mark 121 in response to an operation instruction for the cursor mark 121 (for example, moving the cursor by dragging the mouse); and controlling the display apparatus to display the video information and/or the medical parameter information at a time point which corresponds to an updated position of the cursor mark 121, so as to facilitate the user to view a state of the target object at that time point through the video information, and analyze the medical condition of the patient in combination with the medical parameter information at that time point.

In some embodiments, as shown in FIG. 12, the review interface also displays a video review button 123. The method of this disclosure also includes: controlling the display apparatus to display a cursor mark 121 on the review interface; in response to an operation instruction for the video review button 123, controlling the display apparatus to display video information or video screenshot(s) corresponding to the cursor mark 121, wherein the video information corresponding to the cursor mark 121 can be video information before or within a preset time period before and after a time point which time point corresponds to the cursor mark 121. The preset time period can be reasonably set according to actual requirements and is not specifically limited here.

It is worth mentioning that an executor of the method for processing a medical parameter and video of this disclosure can be a medical device, a medical device server or a workstation display device in the aforementioned medical system, or it can also be implemented by one or more devices in the medical system together, and no specific limitation is made here.

In summary, according to the method for processing a medical parameter and video of this disclosure, after identifying the target event occurring in the video, the medical parameter information within a preset time period before or before and after the target event occurs and the video information when the target event occurs are correspondingly stored with the target event. This can make it more convenient for a user to review the target event, the medical parameter information within a preset time period before or before and after the target event occurs, and the video information when the target event occurs, which helps the user to analyze the data. When a false alarm occurs due to a target event affects a precise measurement of the medical parameter of the medical device, a user can determine whether the alarm is caused by a body movement of the patient through the video information when the target event occurs, so that the user can temporarily block some possible false alarms to reduce alarm fatigue.

Furthermore, this disclosure also provides a medical display device. The medical display device 100 in the embodiment of this disclosure includes but is not limited to a medical device, a central station, a workstation display device, any suitable PC, etc. The following embodiments mainly illustrate this disclosure by taking the medical display device as a workstation display device as an example.

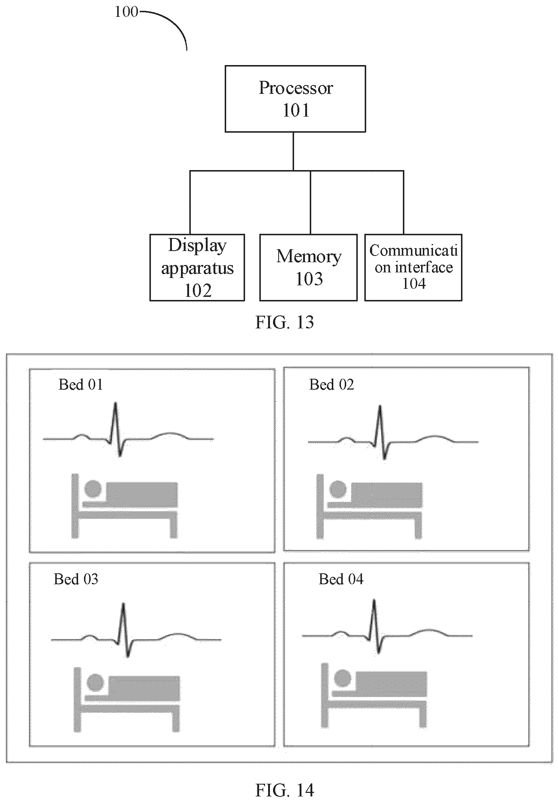

As an example, as shown in FIG. 13, the medical display device 100 includes one or more processors 101, a display apparatus 102, a memory 103, a communication interface 104, and the likes. These components are interconnected via a bus system and/or other forms of connection mechanisms (not shown). It should be noted that components and structures of the medical display device 100 shown in FIG. 13 are merely exemplary and non-restrictive, and the medical display device 100 may also have other components and structures as required.

The memory 103 is configured to store various data and executable programs, such as a system programs for medical display device, various application programs or algorithms for implementing various specific functions, and medical parameter information acquired by a medical device, video information acquired by an imaging apparatus, and video analysis result(s).

The memory 103 may include one or more computer program products, which may include various forms of computer-readable storage media, such as a volatile memory and/or a non-volatile memory. The volatile memory may include, for example, a random access memory (RAM) and/or a cache memory (cache), and the likes. The non-volatile memory may include, for example, a read-only memory (ROM), a hard disk, a flash memory, and the likes. When the medical display device obtains data information transmitted by various devices, such as a monitor, a ventilator, an ultrasound device, and an imaging apparatus, if there is data that needs to be stored locally, it can be stored in the memory 103.

The processor 101 can be a central processing unit (CPU), a graphics processing unit (GPU), an application-specific integrated circuit (ASIC), a field programmable gate array (FPGA), or other forms of processing units with data processing capabilities and/or instruction execution capabilities, and can control other components in the medical display device to perform desired functions. For example, the processor can include one or more of: an embedded processor, a processor core, a microprocessor, a logic circuit, a hardware finite state machine (FSM), a digital signal processor (DSP, a graphics processing unit (GPU), or combination(s) thereof.

In one example, the medical display device 100 also includes a communication interface 104, which is used for communication between various components in the medical display device, and between various components of the medical display device and other devices outside the medical display device (such as imaging apparatuses, medical device servers, medical devices, etc.), so as to exchange information therebetween. The information interaction method between various devices can refer to the relevant description of the medical system in the previous article.

The communication interface 104 can be an interface of any currently known communication protocol, such as a wired interface or a wireless interface, wherein the communication interface 104 can include one or more of: a serial port, a USB interface, an Ethernet port, Wi-Fi, a wired network, a DVI interface, a device integrated interconnect module, or another suitable various port, interface, or connection. The medical display device 100 can also access a wireless network based on a communication standard, such as Wi-Fi, 2G, 3G, 4G, 5G, or a combination thereof. In an exemplary embodiment, the communication interface 104 receives a broadcast signal or broadcast-related information from an external broadcast management system via a broadcast channel. In an exemplary embodiment, the communication interface 104 also includes a near field communication (NFC) module to facilitate short-range communication. For example, the NFC module may be implemented based on a radio frequency identification (RFID) technology, an infrared data association (IrDA) technology, an ultra-wideband (UWB) technology, a Bluetooth (BT) technology, and other technologies.

In one example, when multiple target objects exist and each target object corresponds to one or more medical devices, the communication interface 104 is in direct or indirect communicative connection with the medical device(s) corresponding to each target object, and the medical display device 100 can obtain medical parameter information of the target object acquired by each medical device through the communication interface 104. For example, when the medical device is a monitor, the medical parameter information includes vital sign information. When multiple target objects exist and each target object corresponds to one or more imaging apparatus, the medical display device 100 can acquire video information from each imaging apparatus through the communication interface 104.

In one example, the medical display device 100 also includes an input device (not shown), which may be a device used by a user to input instructions, and may include one or more of a keyboard, a trackball, a mouse, a microphone, a touch screen, etc., or an input device consisting of other control buttons. For example, a user may input instructions for viewing various data information through an input device.

The medical display device 100 of the embodiment of this disclosure also includes an output device, which can output various information (such as images or sounds) to the outside (such as a user), and can include one or more of: a display apparatus 102, a speaker, and the likes.

In one example, the medical display device 100 also includes one or more display apparatuses 102. The display apparatus 102 may be a touch screen, a liquid crystal display, etc., built into the medical display device 100, or an independent display device, such as a liquid crystal display, a television, etc., that is independent of the medical display device 100, or a display screen on an electronic device, such as a mobile phone or a tablet computer, and so on.

The display apparatus 102 is at least configured to display vital sign information and ultrasound information of one or more target objects. The display apparatus 102 can display the vital sign information and the ultrasound information acquired by the processor 101. The medical display device 100 also includes a user interface, and a user of the medical display device can control an operation of the medical display device through the user interface. The user interface can be provided by the display apparatus 102, which may include a touch screen that allows the user to input an operating instruction to the medical display device through the display apparatus 102, and/or include one or more control panels, etc., through which the user can control an operation of the medical display device.

In one example, the processor 101 is configured to execute a program stored in the memory 103, so as to enable the processor 101 to perform a following action: controlling the display apparatus to display an observation interface, wherein the observation interface includes at least one display area, each display area correspondingly displays at least part of the medical parameter information of one target object. For example, the display observation interface shown in FIG.14 provides four display areas, which simultaneously display at least part of medical parameter information of four target objects, such as vital sign information.

Furthermore, a user can also open a detail interface of a corresponding selected target object by operating one of the multiple display areas, for example, by using a mouse or a touch screen. The detail interface can be used to display real-time information and review information of the selected target object. Specifically, the processor 101 is further configured to: when a selection instruction for one of the multiple display areas is obtained (for example, based on clicking the corresponding display area through a mouse or a touch screen), control the display apparatus 102 to display a detail interface of a selected target object which corresponds to the selected display area, wherein the detail interface is provided with a review button (not shown). Optionally, the detail interface is also provided with a view button for real-time information (not shown). Optionally, the detail interface can be located on one side of the display observation interface and does not cover the multiple display areas displayed by the display observation interface, so that while viewing the selected target object, some medical parameter information of other target objects, such as vital sign information, can also be viewed.

In addition to the view button for real-time information and the review button, the detail interface may also have a corresponding button for patient management, so as to switch between a real-time information interface, a review interface, a patient management interface, and the likes, through these buttons. It should be noted that real-time and review are two relative concepts. Exemplarily, the detail interface may be provided with multiple review buttons, which are respectively used to present review information from different review angles.

In one example, the processor 101 is further configured to: when a view instruction for a view button for real-time information is obtained, control the display apparatus 102 to display a real-time interface of the selected target object on the detail interface, and the real-time interface is configured to display real-time information of the selected target object, such as real-time medical parameter information (such as vital sign parameter information) and real-time video information.

In one example, the processor 101 is further configured to: when a review instruction is obtained, control the display apparatus to display a review interface of the selected target object, wherein the review interface is configured to display review information of the selected target object, the review information includes medical parameter information and video information of the target object before a current moment, wherein the video information and the medical parameter information are associated with each other according to a timestamp, so as to enable a user to more conveniently view and analyze the video information and the medical parameter information, thus improving a view and analysis efficiency, and being less prone to errors. In addition, both the medical parameter information and the video information are displayed through the medical display device. Compared with the method in which the video information is displayed through the video browsing terminal and the medical parameter information is displayed through another clinical workstation, this disclosure can save space. When a medical device alarm occurs, a user does not need to manually search for video information of a corresponding target object on another screen, and can confirm a patient state through the video information displayed on the medical display device. It is more convenient, quicker, and less prone to errors.

It is worth mentioning that, the information being stored according to a timestamp means the information is stored according to a time when it was generated, so that the user can retrieve the information within the desired preset time period when reviewing.

In one example, a detail interface of a selected target object is provided with a video access button. When a review interface of the selected target object is displayed on the detail interface, the processor is further configured to: when a video view instruction for the video access button is obtained, obtain historical video information of the selected target object within a preset time period, and control the display apparatus to display the historical video information of the selected target object, thereby facilitating the user to view the state of the target object within the preset time period. The preset time period can be reasonably set according to user requirements, and can also be the same as or different from the preset time period of the aforementioned review information. The historical video information may be displayed in a video display window provided by the display apparatus 102. The video display window may be displayed in a form of a floating window on a current interface of the detail interface, or may be displayed in a review interface of the target object of interest. Optionally, the display apparatus 102 may also provide a control element for controlling a playback speed of the historical video information, a control element for dragging and playing the historical video information, and the likes.

In one example, the detail interface of the selected target object is provided with a video access button. When a real-time interface of the selected target object is displayed on the detail interface, the processor is further configured to: when a video view instruction for the video access button is obtained, obtain current real-time video information of the selected target object, and control the display apparatus to display the current real-time video information of the selected target object. Wherein the real-time video information can be displayed in a real-time video display window provided by the display apparatus 102. The real-time video display window can be displayed in a form of a floating window on a current interface of the detail interface, or can also be displayed in the real-time interface of the target object of interest, etc.

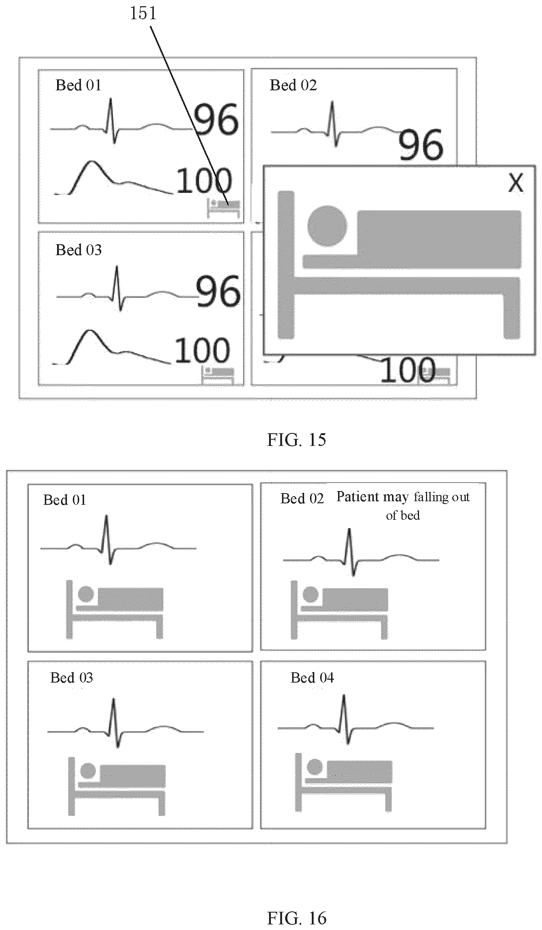

In one example, as shown in FIG. 15, each display area is provided with a video access button 151, and the processor is further configured to: when a video view instruction for the video access button 151 is obtained, obtain current real-time video information of the target object, which target object corresponds to a display area where the video access button 151 is located, and control the display apparatus to display said real-time video information. Alternatively, the observation interface is provided with a video access button; the processor is further configured to: when a video view instruction for the video access button is obtained, obtain current real-time video information of one or more target objects, and control the display apparatus to simultaneously display said real-time video information of the one or more target objects.

In one example, as shown in FIG. 14, each display area displays at least respective part of the medical parameter information and the real-time video information of a target object. This presentation method allows a clinician to simultaneously view the medical parameter information and the real-time video information of multiple target objects.

In one example, each display area displays at least part of the medical parameter information and video screenshot(s) of a target object. The video screenshot(s) is(are) updated every a predetermined period. The predetermined period can be reasonably set according to actual requirements. For example, the video screenshot(s) is(are) updated every 5 seconds, thereby saving a storage space occupied by a real-time display of video information and reducing a network bandwidth occupied by data transmission.

In one example, each display area is further configured to display at least part of the medical parameter information of one target object and event information of a target event, and the target event is obtained by identifying the video information of each target object. For example, as shown in FIG. 16, when a target object which corresponds to any one display area is identified to be in a target event, the display area, which corresponds to the target object in the target event, displays at least part of the medical parameter information and event information of the target event. For example, in FIG. 16, the patient in bed 02 is in a target event that the patient may fall out of bed, then a text representation of "the patient falling out of bed" is displayed in a display area which corresponds to bed 02, wherein the event information of the target event can be displayed in a sub-area of the corresponding display area, which does not overlap with the displayed medical parameter information, or in some embodiments, the event information of the target event can also be displayed in the form of a pop-up window, for example, the event information includes but is not limited to at least one of: text representation of the target event, time information when the target event occurs, and the likes. In some embodiments, the event information may also include video information or image information when the target event occurs, and/or video information within a preset time period before and after the target event occurs. Optionally, the pop-up window may partially cover the corresponding display area, or the pop-up window may cover a partial area of the display observation interface.

In some embodiments, event information of a target event can also be displayed in a preset display mode. For example, the event information is a text representation of the target event. The preset display mode can be different from a display mode of the displayed medical parameter information, such as in a different background color (a red background can be used for that "the patient may fall out of bed" as shown in FIG. 16), in a different font, and or in a flashing manner, or be accompanied by output sound and/or light prompts. In this way, medical staff can be reminded in time that the target object is in the target event so that they can be helped or treated in time.

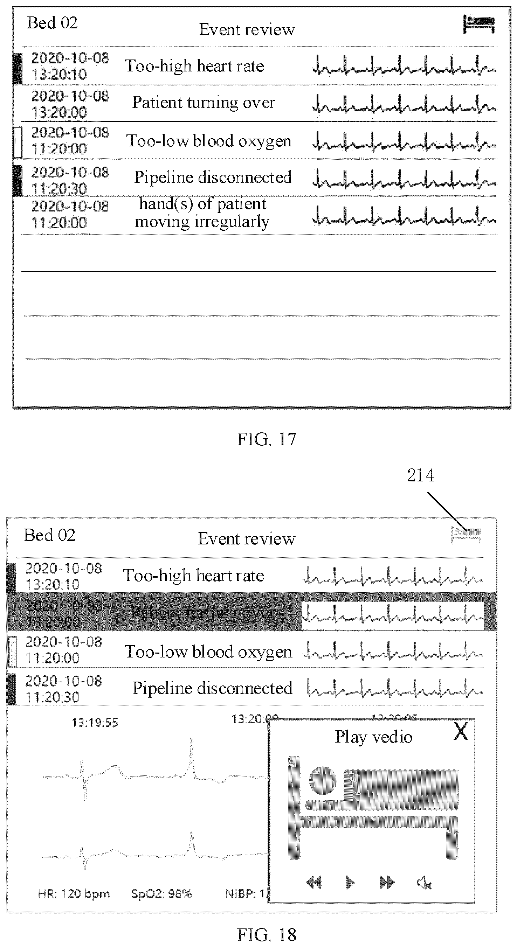

In some embodiments, as shown in FIG. 17, the review information also includes an alarm event list of the selected target object, wherein the alarm event list is for displaying alarm event information of the selected target object within a preset time period, wherein the alarm event information includes a type of alarm event, optionally, the type of alarm event includes at least one of: a target event, an abnormal alarm for a vital sign, and a device abnormal alarm; wherein the target event is obtained by identifying the video information of each target object acquired by an imaging apparatus, and includes at least one of: facial appearance, a movement posture of body part, wherein the movement posture of body part includes at least one of: the target object falling out of bed, the target object turning over, hand(s) of the target object moving irregularly, or other movement postures that may put the target object in danger. Through the alarm event list, medical staff can conveniently review the various events of the target object within the preset time period.

In one example, the alarm event information also includes at least one of: medical parameter information when an alarm event occurs, medical parameter information within a preset time period before and after an alarm event occurs, video information within a preset time period before and after an alarm event occurs, image information when an alarm event occurs, or other suitable information.

In some embodiments, the alarm event list is arranged in a chronological order of respective occurrence time of alarm events, so as to facilitate the user to find the event corresponding to the time point wanted to query in a timely manner.

In one example, multiple alarm events can be marked in different manners, such as with different colors, etc., or different alarm types correspond to identifications with different display manners, and different alarm levels correspond to identifications with different display manners. For example, the alarm levels can include following levels from high to low in terms of alarm urgency: critical, serious, technical, etc., and different alarm levels correspond to identifications with different display methods, such as identifications with different colors.

In one implementation, as shown in FIG. 18, the processor is further configured to: in response to a user instruction to view detailed information of a first alarm event in the alarm event list, control the display apparatus to display the detailed information of the first alarm event; in response to a video review instruction inputted by a user (for example, in response to a video review instruction inputted by a user through the video review button 214), control the display apparatus to display, on a detail interface of the first alarm event, the video information of the target object which information corresponds to a preset time period before or before and after the first alarm event occurs, wherein an occurrence time of the first alarm event is a time point within said preset time period. The video review button 214 may be set at any suitable position, for example, at a top side, left side or other suitable position of the detail interface of alarm event. In this disclosure, various display interfaces and display windows can be reasonably arranged according to actual requirements, and no specific limitation is made here.

The detailed information of the first alarm event includes medical parameter information (such as vital sign parameter information) displayed in at least one type of a trend graph, a trend table, and a waveform graph.

The detailed information of the first alarm event includes, but is not limited to, an occurrence time of alarm, an alarm setting, and vital sign parameter information, a waveform graph and a trend graph, etc., when alarming.

Optionally, the preset time period includes an occurrence time of the first alarm event, a first time period before an occurrence time of the first alarm event, a second time period after an occurrence time of the first alarm event, wherein a total time period of the preset time period can be reasonably set as needed, and the respective time period of the first time period and the second time period can be adjusted arbitrarily. Optionally, the preset time period may include 10s, 20s, 30s, 40s, etc.

In one example, video review information within a preset time period is displayed in a video display window, wherein the video display window also displays a timeline and a play button, the processor 101 is further configured to: in response to a time adjustment instruction of a user for the time line, control the display apparatus 102 to display the video information which corresponds to a time point indicated by the time adjustment instruction, wherein the user can drag an indication of the time line or click the time line, so as to drag and play the video, etc. The user may use the play button to play, pause, or fast forward the video information.

For example, the medical parameter information may be vital sign parameter information of the same target object monitored by a monitor, and the vital sign parameter information includes waveform(s) and/or value(s), trend graph(s), etc., of vital sign parameter(s).

In some embodiments, the processor 101 is further configured to: when a review instruction for review information within a preset time period is obtained, control the display apparatus to display corresponding review information on the review interface according to a time sequence within said preset time period; wherein the review interface displays a time indication which represents the time sequence, and a target event indication is provided at a time point of the time indication which time point corresponds to a target event. In this way, the user can be reminded that a target event has occurred to the target object at the time point indicated by the corresponding target event indication.

In some embodiments, the processor 101 is further configured to: when a selection instruction for one of the multiple display areas is obtained, control the display apparatus to display a detail interface of a selected target object which target object corresponds to a selected display area, wherein the detail interface is provided with a review button; and when a review instruction for review information within a preset time period is obtained, control the display apparatus to display corresponding review information on the review interface according to a time sequence within said preset time period. As shown in FIG. 11, wherein a time indication which indicates the time sequence is displayed on the review interface, a target event indication 122 is provided at a time point of the time indication, which time point corresponds to the target event. In this way, the user can be clearly reminded that a target event has occurred in the target object at the time point indicated by the corresponding target event indication.

In some embodiments, as shown in FIG. 11, the processor 101 is further configured to: when obtaining a selection instruction for one target event indication, control the display apparatus to display event information of the target event which target event corresponds to a selected target event indication (for example, 14:20 both hands move), and display the medical parameter information within a preset time period before or before and after the target event occurs (that is, data information of medical parameter, such as vital sign parameter information). Optionally, the selection instruction may be an instruction generated by moving a cursor to an occurrence time point of the target event, or an instruction generated by clicking the target event indication with a mouse, or other selection instruction for the target event indication. The event information includes but is not limited to at least one of: a text representation of the target event, time information when the target event occurs, and the likes. In some embodiments, the event information may also include video information or image information when the target event occurs, and/or video information within a preset time period before and after the target event occurs. By a combined display of the medical parameter information and the target event, it is convenient for the user to analyze an abnormality of the medical parameter information and determine whether the abnormality of the medical parameter information is caused by the target event, thereby more accurately determining the medical condition of patient.

In one embodiment, as shown in FIG. 11, the review interface also displays a video review button 123. The processor 101 is further configured to: in response to an operation instruction for the video review button 123, control the display apparatus to display, on the review interface, video information within a preset time period before and after the target event occurs.

In some embodiments, as shown in FIG. 12, the review interface also displays a cursor mark 121, and the processor 101 is further configured to: control the display apparatus to display a cursor mark 121 on the review interface, update a position of the cursor mark 121 in response to an operation instruction for the cursor mark 121 (for example, moving the cursor by dragging the mouse); and control the display apparatus to display the video information and/or the medical parameter information at a time point which corresponds to an updated position of the cursor mark 121, so as to facilitate the user to view a state of the target object at that time point through the video information, and analyze the condition of the patient in combination with the medical parameter information at that time point.

In some embodiments, as shown in FIG. 12, the review interface also displays a video review button 123, and the processor 101 is further configured to: control the display apparatus 102 to display a cursor mark 121 on the review interface; in response to an operation instruction for the video review button 123, control the display apparatus to display video information or video screenshot(s) corresponding to the cursor mark 121, wherein the video information corresponding to the cursor mark 121 may be video information before or within a preset time period before and after a time point which corresponds to the cursor mark 121. The preset time period may be reasonably set according to actual requirements, and is not specifically limited here.

The medical display device of the embodiment of this disclosure can combine medical parameter information and video information, such as video data, for a centralized display, for example, vital sign information is combined with patient state information at the same time point, such as facial appearance, expression, physical condition, etc. at that moment, so as to enable a doctor to more conveniently obtain information on the patient in multiple dimensions and make timely diagnosis of the condition based on this information.

In summary, according to the medical display device of this disclosure, review information of a selected target object can be displayed, which review information includes medical parameter information and video information of the target object before a current moment, wherein the video information and the medical parameter information are associated with each other according to a timestamp, so as to enable the user to more conveniently view and analyze the video information and medical parameter information, thereby improving a view and analysis efficiency and being less prone to errors. In addition, both the medical parameter information and the video information are displayed through the medical display device. Compared with a method in which the video information is displayed separately through the video browsing terminal and the medical parameter information is displayed through another clinical workstation, this disclosure can save space. When a medical device alarm occurs, the user does not need to manually search for video information of a corresponding target object on another screen, and can confirm a patient state through the video information displayed on the medical display device. It is more convenient, quicker, and less prone to errors.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, in addition to mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and Ordinary technical personnel in this field can design alternative embodiments without departing from the scope of the attached claims. In the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

## Claims

1. A medical system, **characterized in that**, comprising: an imaging apparatus, a medical device, a video processing server, a medical device server, and a workstation display device;
the imaging apparatus is configured to acquire video information of a target object;
the medical device is configured to acquire medical parameter information of the target object;
the video processing server is in direct or indirect communicative connection with the imaging apparatus, and is configured to obtain the video information of the target object which is acquired by the imaging apparatus, and analyze the video information, so as to obtain and output a video analysis result of the target object;
the medical device server is in direct or indirect communicative connection with the medical device and the video processing server respectively, wherein the medical device server is configured to: obtain the medical parameter information of the target object which is transmitted by the medical device, obtain the video analysis result of the target object which is outputted by the video processing server, and fuse the video analysis result of the target object and the medical parameter information of the target object, so as to obtain and output a fusion result;
the workstation display device is in direct or indirect communicative connection with the medical device server, and is configured to obtain and display the fusion result on a same screen.

2. The medical system according to claim 1, **characterized in that**, the video processing server is further configured to transmit the video analysis result to the workstation display device; the workstation display device is further configured to display, on the same screen, the video analysis result of the target object and the medical parameter information of the target object, wherein the target object is associated with the medical device and the imaging apparatus which correspond to each other.

3. The medical system according to claim 1, **characterized in that**, fusing the video analysis result of the target object and the medical parameter information of the target object, so as to obtain and output a fusion result, comprises:
based on a corresponding relationship between the medical device and the imaging apparatus, fusing the video analysis result of the target object and the medical parameter information of the target object, so as to obtain and output the fusion result, wherein the target object is associated with the medical device and the imaging apparatus which correspond to each other; wherein the fusion result comprises at least one of: the medical parameter information when an alarm event occurs, the medical parameter information before an alarm event occurs or within a preset time period before and after an alarm event occurs, the video information within a preset time period before and after an alarm event occurs, and image information when an alarm event occurs.

4. The medical system according to claim 1, **characterized in that**, the medical device server is further in direct or indirect communicative connection with the imaging apparatus, the workstation display device is further in direct or indirect communicative connection with the imaging apparatus; wherein the workstation display device is further configured to obtain the video information which is transmitted by the imaging apparatus or the medical device or the video processing server or the medical device server, and to obtain the medical parameter information which is transmitted by the medical device server or the medical device, and to display, on the same screen, the medical parameter information and the video information which respective information corresponds to a same target object.

5. The medical system according to any one of claims 1~4, **characterized in that**, the video processing server comprises a first video server and a second video server; wherein the first video server is configured to obtain the video information of the target object which is acquired by the imaging apparatus, and analyze the video information, so as to obtain and output the video analysis result of the target object; the second video server is configured to obtain the video information of the target object through the imaging apparatus, and store and/or forward the video information of the target object.

6. The medical system according to claim 5, **characterized in that**, the second video server is in direct communicative connection with the imaging apparatus, or the second video server is in indirect communicative connection with the imaging apparatus through the medical device; the second video server is further in direct communicative connection with the first video server, and is configured to forward to the first video server the video information of the target object which is acquired by the imaging apparatus;
the first video server and the medical device server are in indirect communicative connection with each other through the medical device or through the second video server; or the first video server and the medical device server are in direct communicative connection with each other.

7. The medical system according to claim 5, **characterized in that**, at least two of the second video server, the first video server and the medical device server are implemented by a same server.

8. The medical system according to claim 5, **characterized in that**, the workstation display device and at least two of the second video server, the first video server and the medical device server are implemented by a same computing device.

9. The medical system according to claim 1, **characterized in that**, the medical device server is further configured to store configuration information, wherein the configuration information comprises a mapping relationship between identification information of the target object and identification information of the medical device, and a mapping relationship between the identification information of the target object and identification information of the imaging apparatus;
the medical device server is further configured to: in response to a retrieving instruction for the video information and/or a retrieving instruction for the medical parameter information; transmit, based on the configuration information, the video information and/or the medical parameter information which respective information corresponds to the retrieving instruction(s) to the workstation display device for display, wherein the retrieving instruction(s) indicates(indicate) the identification information of the target object.

10. The medical system according to claim 9, **characterized in that**, the identification information of the target object comprises at least one of: identity of the target object and a bed number of the target object, the identification information of the medical device comprises a device identity code and/or a device address, and the identification information of the imaging apparatus comprises a corresponding access address for video data of the imaging apparatus.

11. The medical system according to claim 9, **characterized in that**, the medical device server is further configured to: in response to the medical device or the imaging apparatus being replaced, update the configuration information according to identification information of a replaced medical device or identification information of a replaced imaging apparatus.

12. The medical system according to claim 1, **characterized in that**, the medical device server is further configured to store the medical parameter information.

13. The medical system according to claim 1, **characterized in that**, the video processing server is further configured to store the video information of the target object, and the video processing server is stored with video configuration information, wherein the video configuration information is for recording: bed information which corresponds to the target object at any time, a corresponding access address for video data of the imaging apparatus, and time information which corresponds to the video information.

14. The medical system according to claim 1, **characterized in that**, the medical device comprises at least one of: a monitor, an infusion pump, a ventilator and an anesthesia machine; wherein when the medical device is a monitor, the medical parameter information comprises vital sign information of the target object.

15. The medical system according to claim 1, **characterized in that**, the video analysis result is obtained by identifying and processing the video information of each target object which information is acquired by the imaging apparatus, wherein the video analysis result comprises at least one of: facial appearance and movement posture of body part.

16. A medical system, **characterized in that**, comprising: an imaging apparatus, a medical device, a video processing server, a medical device server, and a workstation display device;
the imaging apparatus is configured to acquire video information of a target object;
the medical device is configured to acquire medical parameter information of the target object;
the video processing server is in direct or indirect communicative connection with the imaging apparatus, and is configured to obtain the video information of the target object which is acquired by the imaging apparatus, analyze the video information, so as to obtain and output a video analysis result of the target object;
the medical device is in direct or indirect communicative connection with the video processing server, and is configured to obtain the video analysis result of the target object which is outputted by the video processing server, and fuse the video analysis result of the target object and the medical parameter information of the target object, so as to obtain and output a fusion result;
the medical device server is in communicative connection with the medical device, and is configured to obtain the fusion result which is forwarded by the medical device;
the workstation display device is in direct or indirect communicative connection with the medical device server, and is configured to obtain and display the fusion result on a same screen.

17. A medical system, **characterized in that**, comprising: an imaging apparatus, a medical device, a medical device server and a workstation display device;
the medical device is configured to acquire medical parameter information of a target object;
the imaging apparatus is configured to acquire video information of the target object;
one of the medical device and the imaging apparatus is further configured to analyze the video information, so as to obtain and output a video analysis result of the target object;
the medical device server is in direct communicative connection with the medical device and the imaging apparatus respectively, or the medical device server is in indirect communicative connection with the imaging apparatus; wherein the medical device server is configured to fuse the video analysis result of the target object and the medical parameter information of the target object, so as to obtain and output a fusion result; or the medical device is configured to fuse the video analysis result of the target object and the medical parameter information of the target object, so as to obtain and output a fusion result, and transmit said fusion result to the medical device server;
the workstation display device is in direct or indirect communicative connection with the medical device server, and is configured to obtain and display the fusion result on a same screen.

18. A medical system, **characterized in that**, comprising: an imaging apparatus, a medical device, a medical device server and a workstation display device;
the medical device is configured to acquire medical parameter information of a target object;
the imaging apparatus is configured to acquire video information of the target object; wherein the imaging apparatus is in communicative connection with the medical device;
the medical device server is in direct communicative connection with the medical device, and is configured to obtain the medical parameter information of the target object which is transmitted by the medical device;
the workstation display device is in direct or indirect communicative connection with the medical device server, and is configured to: obtain the video information of the target object which is acquired by the imaging apparatus, analyze the video information to obtain a video analysis result of the target object, obtain the medical parameter information of the target object which is transmitted by the medical device server, and fuse the video analysis result of the target object and the medical parameter information of the target object, so as to obtain and display a fusion result on a same screen.

19. A method for processing a medical parameter and video, **characterized in that**, comprising:
acquiring video information of a target object;
acquiring medical parameter information which is outputted by a medical device which device corresponds to the target object;
after a target event is identified to occur in the video, correspondingly storing the medical parameter information within a preset time period before or before and after the target event occurs, the video information when the target event occurs, and the target event.

20. The method according to claim 19, **characterized in that**, further comprising:
displaying, by a display apparatus, the video information when the target event occurs and the medical parameter information within a preset time period before or before and after the target event occurs.

21. The method according to claim 19, **characterized in that**, the display apparatus provides a display observation interface, wherein the display observation interface comprises multiple display areas, each display area correspondingly displays at least part of the medical parameter information of one target object;
wherein the method further comprises:
when a selection instruction for one of the multiple display areas is obtained, controlling the display apparatus to display a detail interface of a selected target object which target object corresponds to a selected display area, wherein the detail interface is provided with a review button; and
when a review instruction for review information within a preset time period is obtained, controlling the display apparatus to display corresponding review information on a review interface according to a time sequence within said preset time period; wherein the review interface is configured to display a time indication which represents the time sequence; a target event indication is provided at a time point of the time indication which time point corresponds to the target event.

22. The method according to claim 21, **characterized in that**, further comprising:
when a selection instruction for one target event indication is obtained, controlling the display apparatus to display event information of the target event which target event corresponds to a selected target event indication, and display the medical parameter information within a preset time period before or before and after the target event occurs.

23. The method according to claim 21, **characterized in that**, the review interface is further configured to display a cursor mark, and the method further comprises:
after a selection instruction for one target event indication is obtained, controlling the display apparatus to display the cursor mark on the review interface;
in response to an operation instruction for the cursor mark, updating a position of the cursor mark; and
controlling the display apparatus to display the video information and/or the medical parameter information at a time point which corresponds to an updated position of the cursor mark.

24. A medical display device, **characterized in that**, comprising a communication interface, a display apparatus and a processor, wherein:
the communication interface is configured to obtain medical parameter information and video information of at least one target object;
the processor is configured to:
control the display apparatus to display an observation interface, wherein the observation interface comprises at least one display area, wherein each display area correspondingly displays at least part of the medical parameter information of one target object;
when a selection instruction for one of multiple display areas is obtained, control the display apparatus to display a detail interface of a selected target object which target object corresponds to a selected display area;
when a review instruction is obtained, control the display apparatus to display a review interface of the selected target object, wherein the review interface is configured to display review information of the selected target object, wherein the review information comprises the medical parameter information and the video information of the target object before a current moment, wherein the medical parameter information and the video information are associated with each other according to a timestamp.

25. The medical display device according to claim 24, **characterized in that**, the processor is further configured to:
control the display apparatus to display a cursor mark on the review interface;
in response to an operation instruction for the cursor mark, update a position of the cursor mark; and
control the display apparatus to display the video information at a time point which corresponds to an updated position of the cursor mark.

26. The medical display device according to claim 24, **characterized in that**, the detail interface of the selected target object is provided with a video access button, and when the review interface of the selected target object is displayed on the detail interface, the processor is further configured to: when a video view instruction for the video access button is obtained, obtain historical video information of the selected target object within a preset time period, and control the display apparatus to display the historical video information of the selected target object.

27. The medical display device according to claim 24, **characterized in that**, the detail interface of the selected target object is provided with a video access button, and when a real-time interface of the selected target object is displayed on the detail interface, the processor is further configured to: when a video view instruction for the video access button is obtained, obtain current real-time video information of the selected target object, and control the display apparatus to display the current real-time video information of the selected target object.

28. The medical display device according to claim 24, **characterized in that**, each display area is provided with a video access button; wherein the processor is further configured to: when a video view instruction for the video access button is obtained, obtain current real-time video information of the target object which target object corresponds to the display area where the video access button is located, and control the display apparatus to display said real-time video information; or
the observation interface is provided with a video access button; wherein the processor is further configured to: when a video view instruction for the video access button is obtained, obtain current real-time video information of one or more target objects, and control the display apparatus to simultaneously display said real-time video information of the one or more target objects.

29. The medical display device according to claim 24, **characterized in that**, each display area is configured to display at least part of the medical parameter information of one target object and real-time video information of said target object; or
each display area is configured to display at least part of the medical parameter information of one target object and video screenshot(s) of said target object, wherein the video screenshot(s) is(are) updated every predetermined time period.

30. The medical display device according to claim 24, **characterized in that**, each display area is configured to display at least part of the medical parameter information of one target object and event information of a target event; wherein the target event is obtained by identifying the video information of each target object.

31. The medical display device according to claim 24, **characterized in that**, the review information further comprises an alarm event list of the selected target object, wherein the alarm event list is for displaying alarm event information of the selected target object within a preset time period, wherein the alarm event information comprises a type of alarm event.

32. The medical display device according to claim 31, **characterized in that**, the type of alarm event comprises at least one of: a target event, an abnormal alarm for a vital sign, and a device abnormal alarm; wherein the target event is obtained by identifying the video information of each target object which is acquired by an imaging apparatus, and the target event comprises at least one of: facial appearance and movement posture of body part.

33. The medical display device according to claim 32, **characterized in that**, the movement posture of body part comprises at least one of: the target object falling out of bed, the target object turning over, and hand(s) of the target object moving irregularly.

34. The medical display device according to claim 31, **characterized in that**, the alarm event information further comprises at least one of: the medical parameter information when an alarm event occurs, the medical parameter information before an alarm event occurs or within a preset time period before and after an alarm event occurs, the video information within a preset time period before and after an alarm event occurs, and image information when an alarm event occurs.

35. The medical display device according to claim 31, **characterized in that**, the alarm event list is arranged in a chronological order of respective occurrence time of alarm events.

36. The medical display device according to claim 31, **characterized in that**, the processor is further configured to:
in response to a user instruction to view detailed information of a first alarm event in the alarm event list, control the display apparatus to display the detailed information of the first alarm event; and
in response to a video review instruction inputted by a user, control the display apparatus to display, on a detail interface of the first alarm event, the video information of the target object which information corresponds to a preset time period before or before and after the first alarm event occurs, wherein an occurrence time of the first alarm event is a moment within said preset time period.

37. The medical display device according to claim 36, **characterized in that**, the detailed information of the first alarm event comprises the medical parameter information which is displayed in at least one type of: a trend graph, a trend table and a waveform graph.

38. The medical display device according to claim 24, **characterized in that**, the processor is further configured to:
when the review instruction for the review information within a preset time period is obtained, control the display apparatus to display the corresponding review information on the review interface according to a time sequence within said preset time period; wherein the review interface is configured to display a time indication which represents the time sequence; a target event indication is provided at a time point of the time indication which time point corresponds to a target event; wherein the target event is obtained by identifying the video information of each target object which is acquired by an imaging apparatus.

39. The medical display device according to claim 38, **characterized in that**, the processor is further configured to:
when a selection instruction for one target event indication is obtained, control the display apparatus to display event information of the target event which target event corresponds to a selected target event indication, and display the medical parameter information within a preset time period before or before and after the target event occurs.

40. The medical display device according to claim 39, **characterized in that**, the review interface is further configured to display a cursor mark; the processor is further configured to:
when the selection instruction for one target event indication is obtained, control the display apparatus to display the cursor mark which corresponds to the selected target event indication, and display, adjacent to the cursor mark, the event information of the target event and the medical parameter information within a preset time period before or before and after the target event occurs, wherein a position of the cursor mark corresponds to a corresponding occurrence moment of said target event on a timeline.

41. The medical display device according to any one of claims 24-40, **characterized in that**, when the medical display device comprises a monitor, the medical parameter information comprises vital sign information of the target object which is acquired by a corresponding medical device.
